(19) Europäisches Patentamt
European Patent Office
Office européen des brevets

(11) **EP 4 285 815 A1**

(12) **EUROPEAN PATENT APPLICATION**
published in accordance with Art. 153(4) EPC

(43) Date of publication:
**06.12.2023 Bulletin 2023/49**

(21) Application number: **22770906.0**

(22) Date of filing: **01.02.2022**

(51) International Patent Classification (IPC):
*A61B 5/022* (2006.01)    *A61B 5/0205* (2006.01)
*A61B 5/0215* (2006.01)

(52) Cooperative Patent Classification (CPC):
**A61B 5/02028; A61B 5/0205; A61B 5/02208;
A61B 5/0245; A61B 5/7282**

(86) International application number:
**PCT/JP2022/003892**

(87) International publication number:
**WO 2022/196144 (22.09.2022 Gazette 2022/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.03.2021 JP 2021045023**

(71) Applicant: **TERUMO Kabushiki Kaisha
Tokyo 151-0072 (JP)**

(72) Inventors:
• **HATTA, Tomonori**
  **Ashigarakami-gun, Kanagawa 259-0151 (JP)**
• **ICHIKAWA, Ryo**
  **Ashigarakami-gun, Kanagawa 259-0151 (JP)**

(74) Representative: **Casalonga
Casalonga & Partners
Bayerstraße 71/73
80335 München (DE)**

(54) **ARTERIAL PRESSURE ESTIMATION DEVICE, ARTERIAL PRESSURE ESTIMATION SYSTEM, AND ARTERIAL PRESSURE ESTIMATION METHOD**

(57)    An arterial-pressure estimation apparatus includes a control unit configured to acquire sensor data indicating, as a blood flow timing, a timing at which a blood flow generated at one or more locations of a blood vessel downstream of an aorta is detected for each heartbeat when the one or more locations is compressed while a pressure is gradually reduced, record a value of the pressure corresponding to each blood flow timing, correct the value of the pressure corresponding to the blood flow timing included in an inspiration period of a respiration cycle, and estimate a change in an arterial pressure over time by referring to the acquired sensor data and the recorded and corrected value of the pressure.

FIG. 1

EP 4 285 815 A1

## Description

Technical Field

The present disclosure relates to an arterial-pressure estimation apparatus, an arterial-pressure estimation system, and an arterial-pressure estimation method.

Background Art

PTL 1 discloses a technique of estimating a left intracardiac pressure based on a heart sound, an upper arm cuff pressure, and a K sound. The term "K" is an abbreviation for Korotkoff. PTL 2 discloses a technique of calculating a pressure conversion value of a respiratory variation. PTL 3 discloses a technique of determining a relation between an average value of magnitudes of a plurality of arterial waves obtained within a time equal to or longer than a respiration cycle and an actual blood pressure value.

Citation List

Patent Literature

PTL 1: US2015/0265163
PTL 2: JP2016-137087A
PTL 3: JPH01-214338A

Summary of Invention

Technical Problem

The left intracardiac pressure of a heart is an important observation item in a heart failure diagnosis. However, a direct measurement of the left intracardiac pressure requires inserting a device such as a sensor into the heart, which is highly invasive.
It is generally known that a blood pressure fluctuates with respiration. In the technique disclosed in PTL 1, since data is collected over multiple beats, an influence of such a respiratory variation is included in the data, and an arterial pressure waveform estimated using the data cannot be obtained with sufficient accuracy. In the technique disclosed in PTL 2 or PTL 3, it is difficult to improve an accuracy of estimating an arterial pressure waveform.
An object of the present disclosure is to noninvasively and highly accurately estimate a change in an arterial pressure over time.

Solution to Problem

An arterial-pressure estimation apparatus according to an aspect of the present disclosure includes a control unit configured to acquire sensor data indicating, as a blood flow timing, a timing at which a blood flow generated at one or more locations of a blood vessel downstream of an aorta is detected for each heartbeat when the one or more locations is compressed while a pressure is gradually reduced, record a value of the pressure corresponding to each blood flow timing, correct the value of the pressure corresponding to the blood flow timing included in an inspiration period of a respiration cycle, and estimate a change in an arterial pressure over time by referring to the acquired sensor data and the recorded and corrected value of the pressure.
In an embodiment, the control unit corrects the value of the pressure corresponding to the blood flow timing included in the inspiration period by performing weighting according to an elapsed time in the inspiration period.
In an embodiment, the control unit sets a weighting coefficient based on waveform data indicating a blood pressure waveform obtained by performing an invasive examination.
In an embodiment, the sensor data includes data indicating, as the blood flow timing, a timing at which the one or more locations is compressed multiple times while the pressure is reduced at different speeds each time, and the blood flow is detected for each heartbeat in each of the multiple times, and the control unit performs control to match a timing at which the pressure starts to be reduced with the respiration cycle.
In an embodiment, the sensor data includes data indicating, as the blood flow timing, a timing at which the one or more locations is compressed multiple times while the pressure starts to be reduced at different speeds each time and is reduced at a common speed after a heartbeat at which the blood flow is detected, and the blood flow is detected for each heartbeat in each of the multiple times, and the control unit performs control to match a timing at which the pressure starts to be reduced with the respiration cycle.
In an embodiment, the sensor data includes data indicating, as a cardiac output timing, a timing at which a cardiac output is detected for each heartbeat, and the control unit estimates a change in the arterial pressure over time according to a time difference between the cardiac output timing and the blood flow timing indicated by the sensor data and the value of the pressure.
In an embodiment, the control unit estimates an LVEDP based on an estimation result of the change in the arterial pressure over time.
In an embodiment, the control unit estimates an arterial pressure waveform as the change in the arterial pressure over time, estimates a left ventricular pressure waveform according to the estimated arterial pressure waveform, and acquires an estimated value of the LVEDP based on the estimated left ventricular pressure waveform.
In an embodiment, the control unit inputs the estimation result of the change in the arterial pressure over time to a trained model and acquires an estimated value of the LVEDP from the trained model.

In an embodiment, the control unit generates a trained model for acquiring an estimated value of the LVEDP by performing machine learning using at least a part of the estimation result of the change in the arterial pressure over time.

In an embodiment, the control unit presents an estimated value of the LVEDP to a user.

In an embodiment, the control unit estimates a parameter related to intracardiac hemodynamics based on an estimation result of the change in the arterial pressure over time.

In an embodiment, the parameter includes a pulmonary artery pressure or a pulmonary wedge pressure.

An arterial-pressure estimation system according to an aspect of the present disclosure includes: the arterial-pressure estimation apparatus; and a sensor configured to detect the blood flow.

In an embodiment, the arterial-pressure estimation system further includes an inflation portion configured to compress the at least one location.

An arterial-pressure estimation method according to an aspect of the present disclosure includes: an inflation portion compressing one or more locations of a blood vessel downstream of an aorta while a pressure is gradually reduced; a sensor detecting, for each heartbeat, a blood flow generated at the one or more locations; a control unit acquiring sensor data indicating, as a blood flow timing, a timing at which the blood flow is detected for each heartbeat when the one or more locations is compressed; the control unit recording a value of the pressure corresponding to each blood flow timing; the control unit correcting the value of the pressure corresponding to the blood flow timing included in an inspiration period of a respiration cycle; and the control unit estimating a change in an arterial pressure over time by referring to the acquired sensor data and the recorded and corrected value of the pressure.

Advantageous Effect of Invention

According to the present disclosure, the change in the arterial pressure over time can be noninvasively and highly accurately estimated.

Brief Description of Drawings

[Fig. 1] Fig. 1 is a block diagram illustrating a configuration of an arterial-pressure estimation system according to an embodiment of the present disclosure.
[Fig. 2] Fig. 2 is a block diagram illustrating a configuration of an arterial-pressure estimation apparatus according to the embodiment of the present disclosure.
[Fig. 3] Fig. 3 is a graph illustrating an example of a respiratory variation of a blood pressure.
[Fig. 4] Fig. 4 is a diagram illustrating an example of a respiration cycle, a cuff pressure, a blood pressure waveform, a K-sound waveform, and an electrocardiographic waveform according to the embodiment of the present disclosure.
[Fig. 5] Fig. 5 is a graph illustrating an example of plotted points and an estimated curve in the embodiment of the present disclosure.
[Fig. 6] Fig. 6 is a flowchart illustrating an operation of the arterial-pressure estimation system according to the embodiment of the present disclosure.
[Fig. 7] Fig. 7 is a flowchart illustrating a procedure of a measurement process shown in Fig. 6.
[Fig. 8] Fig. 8 is a graph illustrating an example of plotted points in a modification of the embodiment of the present disclosure.
[Fig. 9] Fig. 9 is a flowchart illustrating an operation of an arterial-pressure estimation system according to the modification of the embodiment of the present disclosure.
[Fig. 10A] Fig. 10A is a diagram illustrating an arterial pressure waveform, a left ventricular pressure waveform, and a heart sound waveform in the vicinity of an aortic valve according to a comparative example.
[Fig. 10B] Fig. 10B is a diagram illustrating a blood flow corresponding to Fig. 10A.
[Fig. 11A] Fig. 11A is a diagram illustrating an arterial pressure waveform, a left ventricular pressure waveform, a heart sound waveform, and an arterial pressure waveform of an upper arm in the vicinity of an aortic valve according to a comparative example.
[Fig. 11B] Fig. 11B is a diagram illustrating a blood flow corresponding to Fig. 11A.
[Fig. 12A] Fig. 12A is a diagram illustrating an arterial pressure waveform, a left ventricular pressure waveform, a heart sound waveform, and an arterial pressure waveform of an upper arm in the vicinity of an aortic valve according to a comparative example.
[Fig. 12B] Fig. 12B is a diagram illustrating a blood flow corresponding to Fig. 12A.
[Fig. 13A] Fig. 13A is a diagram illustrating an arterial pressure waveform, a left ventricular pressure waveform, a heart sound waveform, an arterial pressure waveform of an upper arm, an electrocardiographic waveform, and a first K-sound waveform of the upper arm in the vicinity of an aortic valve according to a comparative example.
[Fig. 13B] Fig. 13B is a diagram illustrating a blood flow corresponding to Fig. 13A.
[Fig. 14] Fig. 14 is a diagram illustrating an arterial pressure waveform, a left ventricular pressure waveform, a heart sound waveform, an arterial pressure waveform of an upper arm, an electrocardiographic waveform, and a first K-sound waveform of the upper arm in the vicinity of an aortic valve according to a comparative example.
[Fig. 15] Fig. 15 is a diagram illustrating an arterial pressure waveform, a left ventricular pressure waveform, a heart sound waveform, an arterial pressure waveform of an upper arm, an electrocardiographic waveform, a first K-sound waveform of the upper arm, an estimated arterial pressure waveform, and a fitted curve in the vicinity of an aortic valve according to a comparative example.

[Fig. 16] Fig. 16 is a diagram illustrating an arterial pressure waveform, a left ventricular pressure waveform, a heart sound waveform, an arterial pressure waveform of an upper arm, an electrocardiographic waveform, a first K-sound waveform of the upper arm, an estimated arterial pressure waveform, a fitted curve, and an estimated LVEDP in the vicinity of an aortic valve according to a comparative example.

Description of Embodiments

As comparative examples, similar to the technique disclosed in PTL 1, a method of estimating a left intracardiac pressure based on an arterial pressure waveform will be described with reference to the drawings.

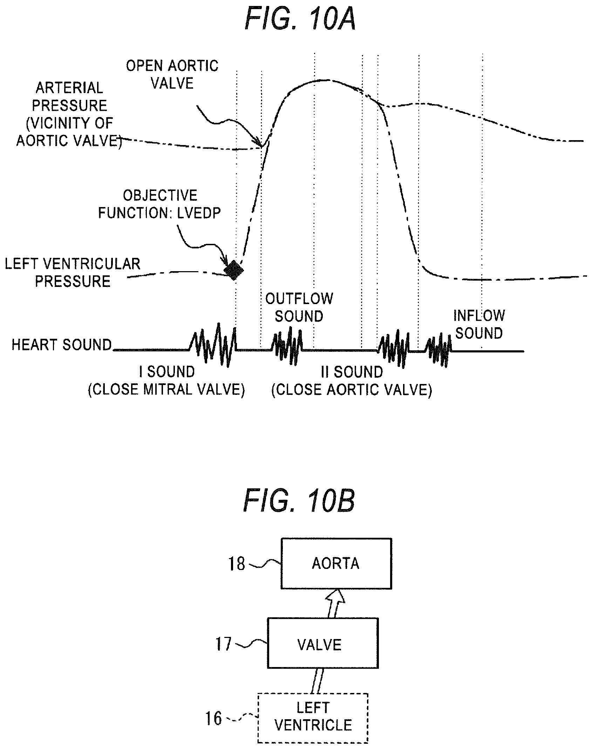

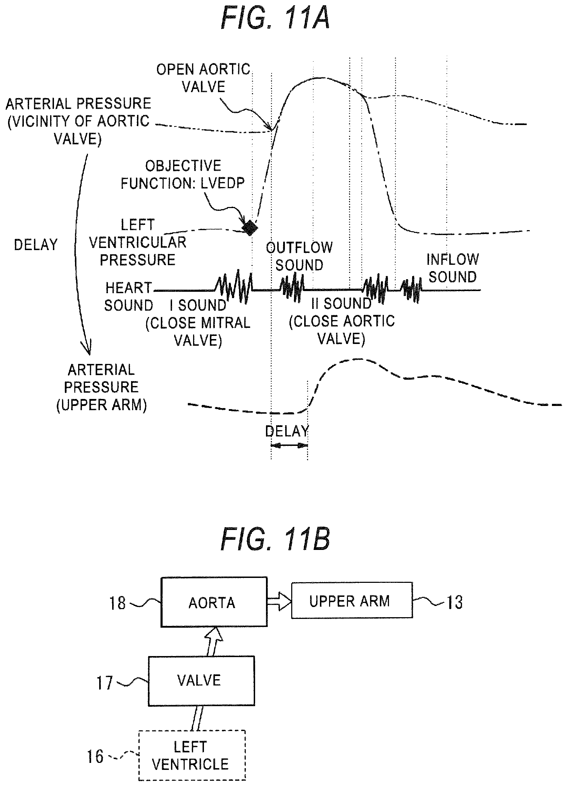

As shown in Figs. 10A and 10B, when a pressure in a left ventricle 16 increases, an aortic valve 17 opens and a blood flows into an aorta 18. As shown in Figs. 11A and 11B, a blood flow passing through the aortic valve 17 reaches an arm 13, which is an upper arm, with a delay. An arterial pressure waveform of the arm 13 is a curve obtained by shifting an arterial pressure waveform in the vicinity of the aortic valve 17 by a delay.

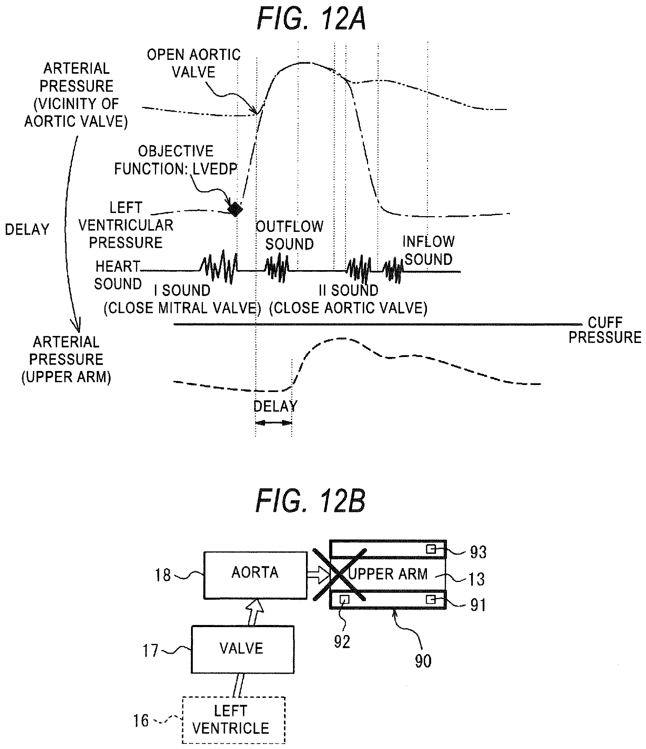

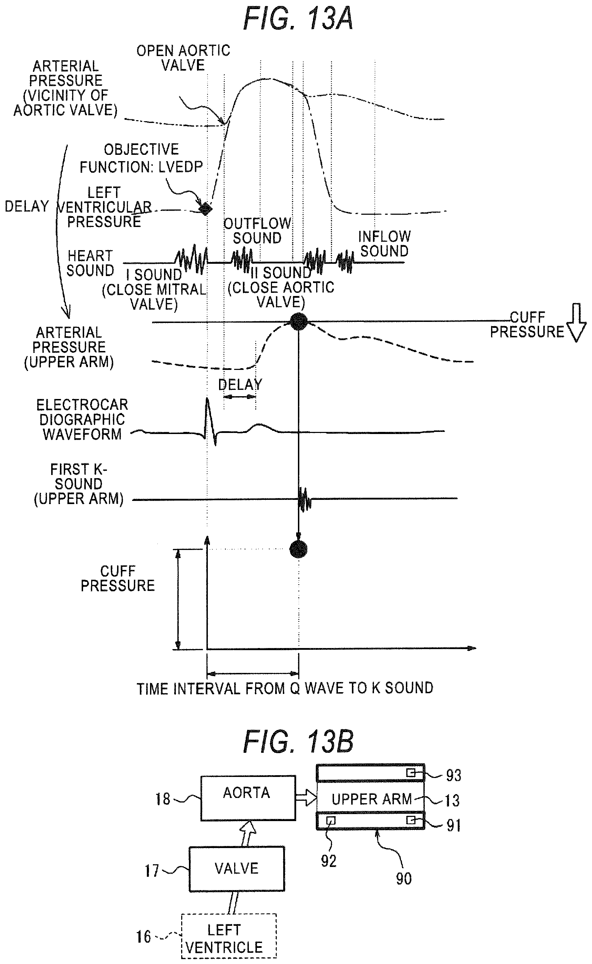

As shown in Figs. 12A and 12B, when a cuff pressure is high, no blood flows into the arm 13. The cuff pressure is a pressure of a cuff 90 attached to the arm 13. A first K-sound microphone 91, a second K-sound microphone 92, and a pressure sensor 93 are incorporated in the cuff 90. As shown in Figs. 13A and 13B, a blood starts to flow into the arm 13 when a cuff pressure is reduced while obtaining an electrocardiographic waveform and waiting for a K sound. When the K sound generated when the blood flows is captured by the first K-sound microphone 91, a time interval from a Q wave to the K sound and a cuff pressure measured by the pressure sensor 93 are recorded. Instead of the time interval from the Q wave to the K sound, a time interval from a heart sound to the K sound may be recorded. A distance between the first K-sound microphone 91 and the second K-sound microphone 92 is known, and is, for example, about 3 cm or more and 5 cm or less. A delay between the aortic valve 17 and the first K-sound microphone 91 is calculated according to a difference between timings at which signals enter the first K-sound microphone 91 and the second K-sound microphone 92 and a distance between the aortic valve 17 and the first K-sound microphone 91.

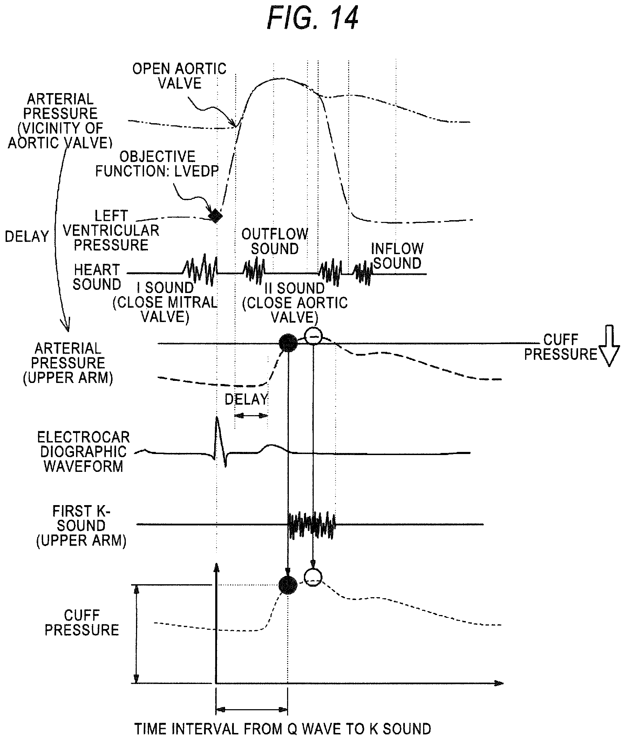

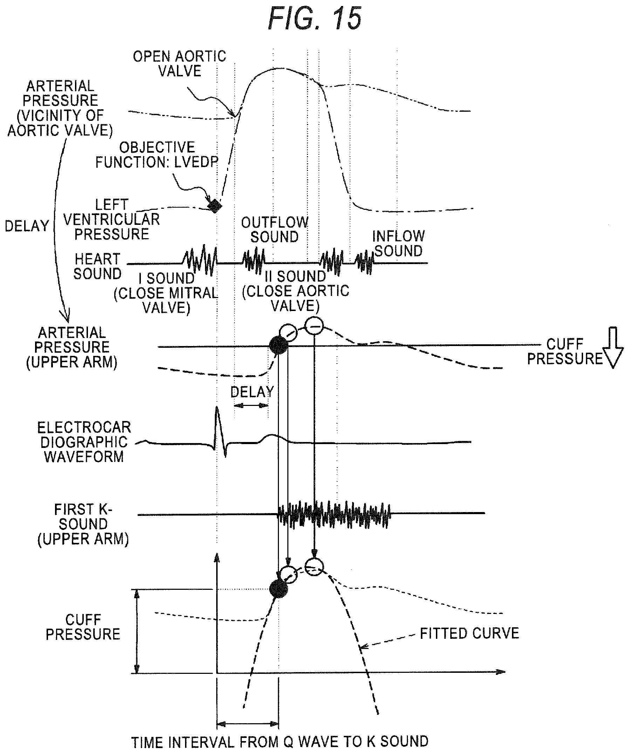

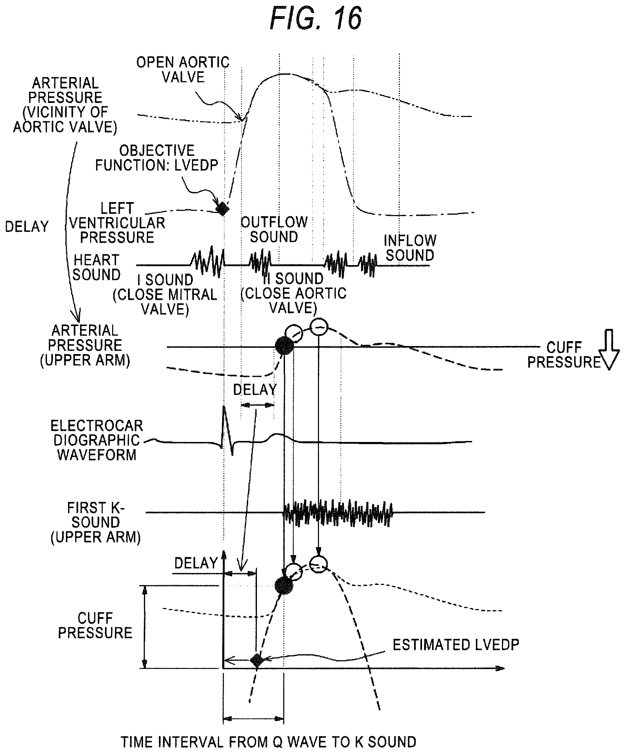

As shown in Fig. 14, when a cuff pressure is further reduced, a start timing of a K sound in subsequent heartbeats is advanced. When the K sound is captured by the first K-sound microphone 91 for each heartbeat, a time interval from a Q wave to the K sound and the cuff pressure measured by the pressure sensor 93 are recorded. Instead of the time interval from the Q wave to the K sound, a time interval from a heart sound to the K sound may be recorded. As shown in Fig. 15, records of a time interval and a cuff pressure are curve fitted into some curve such as a polynomial curve or a cosine curve. It is ideal that the curve has the same waveform as a left ventricular pressure waveform, but this is difficult in practice. As shown in Fig. 16, a pressure value at a time when a delay elapses from a timing of a Q wave is an estimated LVEDP. The term "LVEDP" is an abbreviation for left ventricular end-diastolic pressure.

As described above, in a method in which a cuff pressure is gradually reduced for each heartbeat and acquisition of information on an arterial pressure is completed in multiple beats, a reproducibility of an estimated arterial pressure waveform is not sufficiently obtained due to an influence of a respiratory variation. Therefore, in the present disclosure, the reproducibility of the estimated arterial pressure waveform is improved by correcting a value of the cuff pressure obtained for each beat according to a respiration cycle.

Hereinafter, several embodiments of the present disclosure will be described with reference to the drawings.

In the drawings, the same or corresponding parts are denoted by the same reference numerals. In the description of the embodiments, the description of the same or corresponding parts will be omitted or simplified as appropriate.

An embodiment of the present disclosure will be described.

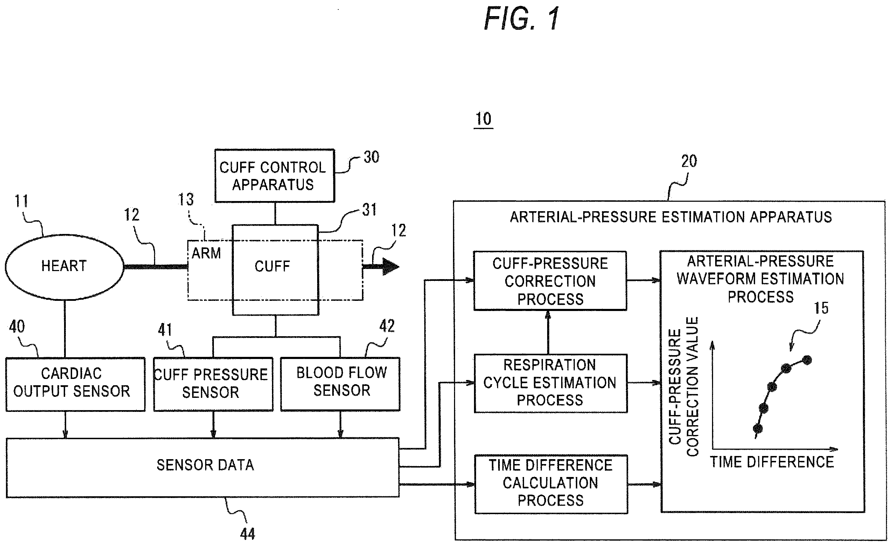

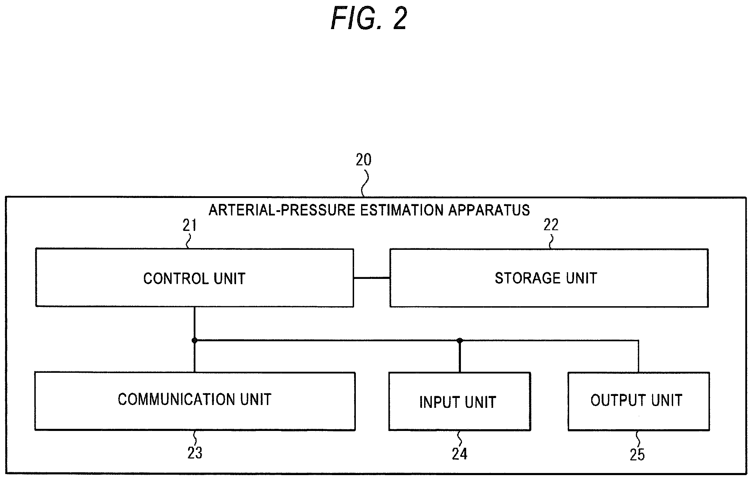

An outline of the present embodiment will be described with reference to Figs. 1 and 2.

In an arterial-pressure estimation system 10 according to the present embodiment, an inflation portion compresses one or more locations of a blood vessel 12 downstream of the aorta 18 while a pressure is gradually reduced. A blood flow sensor 42 detects a blood flow generated at the one or more locations for each heartbeat. A control unit 21 acquires sensor data 44 indicating, as a blood flow timing, a timing at which the blood flow is detected for each heartbeat when the one or more locations is compressed. The control unit 21 records a value of the pressure corresponding to each blood flow timing. The control unit 21 corrects the value of the pressure corresponding to the blood flow timing included in an inspiration period of a respiration cycle. The control unit 21 estimates a change in an arterial pressure over time by referring to the acquired sensor data 44 and the recorded and corrected value of the pressure.

According to the present embodiment, the influence of the respiratory variation can be reduced. Therefore, the change in the arterial pressure over time can be noninvasively and highly accurately estimated.

In the present embodiment, a cuff 31 is used as the inflation portion. The cuff 31 is attached to the arm 13 and compresses one location of the blood vessel 12. The control unit 21 estimates an arterial pressure waveform 15 as the change in the arterial pressure over time. According to the present embodiment, a reproducibility of the estimated arterial pressure waveform 15 can be improved by correcting the value of the cuff pressure obtained for each beat according to the respiration cycle. As a modification of the present embodiment, the inflation portion may be an air bag instead of the cuff 31.

A configuration of the arterial-pressure estimation system 10 according to the present embodiment will be described with reference to Fig. 1.

The arterial-pressure estimation system 10 includes an arterial-pressure estimation apparatus 20, a cuff control apparatus 30, the cuff 31, a cardiac output sensor 40, a cuff pressure sensor 41, and the blood flow sensor 42.

The arterial-pressure estimation apparatus 20 is a computer. The arterial-pressure estimation apparatus 20 is, for example, a dedicated device, a general-purpose device such as a PC, or a server device belonging to a cloud computing system or another computing system. The term "PC" is an abbreviation for personal computer.

The cuff control apparatus 30 is a device that controls the cuff 31. The cuff control apparatus 30 can communicate with the arterial-pressure estimation apparatus 20 directly or via a network such as a LAN or the Internet. The term "LAN" is an abbreviation for local area network.

The cardiac output sensor 40 is a sensor that detects a cardiac output of a heart 11. The cardiac output sensor 40 is an ECG sensor in the present embodiment, and may be a sound sensor that detects a choking sound of a mitral valve. The term "ECG" is an abbreviation for electrocardiogram. The cardiac output sensor 40 can communicate with the arterial-pressure estimation apparatus 20 directly or via a network such as a LAN or the Internet.

The cuff pressure sensor 41 detects a pressure of the cuff 31, that is, a cuff pressure. The cuff pressure sensor 41 can communicate with the arterial-pressure estimation apparatus 20 directly or via a network such as a LAN or the Internet. As a modification of the present embodiment, the cuff pressure sensor 41 may be integrated with the cuff 31.

The blood flow sensor 42 detects a blood flow generated at a location of the blood vessel 12 compressed by the cuff 31. In the present embodiment, the blood flow sensor 42 is a sound sensor that is attached to the arm 13 on a downstream side of the cuff 31 and detects sound generated by a blood flowing downstream of the cuff 31, and may be a PPG sensor or an ultrasonic sensor that measures a blood flow by using an ultrasonic Doppler method. The term "PPG" is an abbreviation for photoplethysmogram. The blood flow sensor 42 can communicate with the arterial-pressure estimation apparatus 20 directly or via a network such as a LAN or the Internet. As a modification of the present embodiment, the blood flow sensor 42 may be integrated with the cuff 31.

The arterial-pressure estimation apparatus 20 acquires, as the sensor data 44, signals output from the cardiac output sensor 40, the cuff pressure sensor 41, and the blood flow sensor 42 when the cuff 31 compresses the one location of the blood vessel 12 while the cuff pressure is gradually reduced under the control of the cuff control apparatus 30. The sensor data 44 is data indicating a cardiac output timing Ti, a pressure Pi, and a blood flow timing Fi for each heartbeat i when i = 1, 2, ..., and n, wherein n is an integer of 2 or more such as 10. The cardiac output timing Ti is a timing at which a cardiac output is detected by the cardiac output sensor 40. The cardiac output timing Ti is treated as a reference timing of the heart 11. The reference timing is a timing that can be specified for each heartbeat i. The pressure Pi is a cuff pressure detected by the cuff pressure sensor 41. The blood flow timing Fi is a timing at which a blood flow is detected by the blood flow sensor 42.

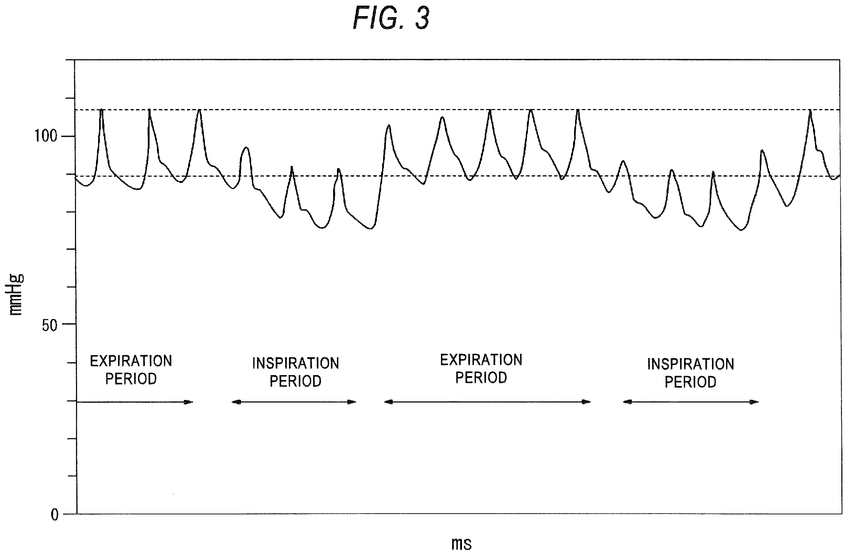

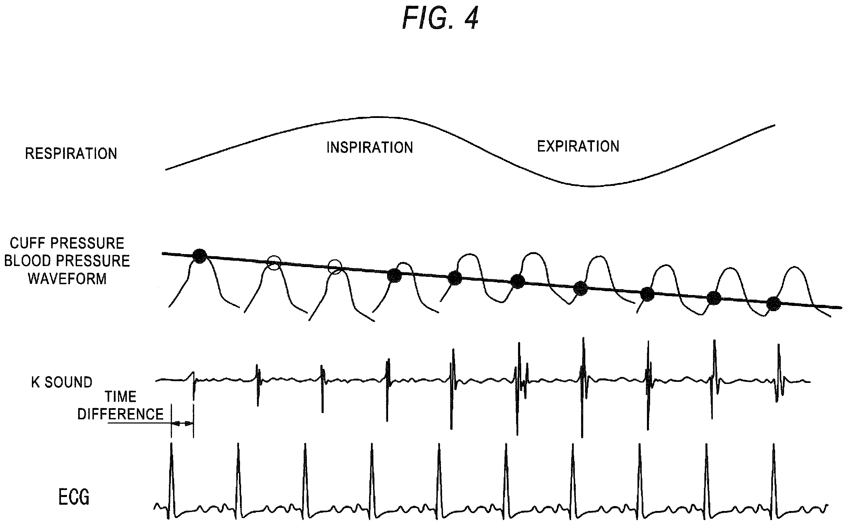

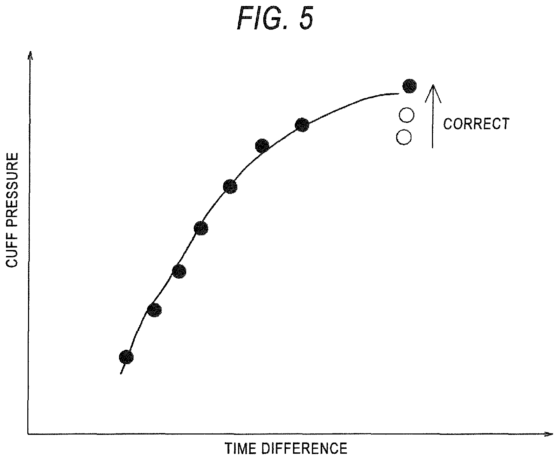

The arterial-pressure estimation apparatus 20 calculates a time difference Di from the cardiac output timing Ti to the blood flow timing Fi, and records a value of the pressure Pi corresponding to the blood flow timing Fi by referring to the sensor data 44. As shown in Fig. 3, a blood pressure waveform fluctuates in a direction in which a blood pressure is reduced during the inspiration period of the respiration cycle. As a result, as shown in Fig. 4, a delay corresponding to the fluctuation occurs in a blood flow timing included in the inspiration period. Therefore, as shown in Fig. 5, the arterial-pressure estimation apparatus 20 corrects a value of a cuff pressure corresponding to the blood flow timing included in the inspiration period.

The arterial-pressure estimation apparatus 20 estimates the arterial pressure waveform 15 based on time differences D1, D2, ..., and Dn and values of corresponding pressures P1, P2, ..., and Pn. A value after correction among the values of the pressures P1, P2, ..., and Pn is used as the value corresponding to the blood flow timing included in the inspiration period. The estimated arterial pressure waveform 15 corresponds to an arterial pressure waveform of the aorta 18. Therefore, the arterial-pressure estimation apparatus 20 estimates an LVEDP based on the estimated arterial pressure waveform 15.

According to present embodiment, it is possible to provide an important determination index in a heart failure diagnosis using an LVEDP value estimated by the arterial-pressure estimation apparatus 20. Based on the index, it is possible to change a prescription of a diuretic or the like, determine hospitalization, determine a hospital visit, or determine discharge from a hospital. With respect to the change of the prescription, a prescription can be issued and a patient can be instructed to take a drug based on data confirmation and determination by a doctor at the time of remote or hospital visit. With respect to the determination of hospitalization, hospital visit, or discharge, a certain threshold may be provided, and when the LVEDP value is an abnormal value, a display suggesting hospitalization or a hospital visit may be performed, and when the LVEDP value is a normal value, a display suggesting a discharge may be performed. When the LVEDP value is an abnormal value, a user or the doctor may be notified of an alert. The arterial-pressure estimation apparatus 20 can also be used for a remote diagnosis.

A configuration of the arterial-pressure estimation apparatus 20 according to the present embodiment will be described with reference to Fig. 2.

The arterial-pressure estimation apparatus 20 includes the control unit 21, a storage unit 22, a communication unit 23, an input unit 24, and an output unit 25.

The control unit 21 includes at least one processor, at least one programmable circuit, at least one dedicated circuit, or any combination thereof. A processor is a general-purpose processor such as a CPU or GPU, or a dedicated processor specialized for a specific process. The term "CPU" is an abbreviation for central processing unit. The term "GPU" is an abbreviation for graphics processing unit. The programmable circuit is, for example, an FPGA. The term "FPGA" is an abbreviation for field-programmable gate array. The dedicated circuit is, for example, an ASIC. The term "ASIC" is an abbreviation for application specific integrated circuit. The control unit 21 executes a process related to an operation of the arterial-pressure estimation apparatus 20 while controlling each unit of the arterial-pressure estimation apparatus 20.

The storage unit 22 includes at least one semiconductor memory, at least one magnetic memory, at least one optical memory, or any combination thereof. The semiconductor memory is, for example, a RAM or a ROM. The term "RAM" is an abbreviation for random access memory. The term "ROM" is an abbreviation for read only memory. The RAM is, for example, an SRAM or a DRAM. The term "SRAM" is an abbreviation for static random access memory. The term "DRAM" is an abbreviation for dynamic random access memory. The ROM is, for example, an EEPROM. The term "EEPROM" is an abbreviation for electrically erasable programmable read only memory. The storage unit 22 functions as, for example, a main storage device, an auxiliary storage device, or a cache memory. The storage unit 22 stores data used for the operation of the arterial-pressure estimation apparatus 20 and data obtained by the operation of the arterial-pressure estimation apparatus 20.

The communication unit 23 includes at least one communication interface. The communication interface is, for example, a LAN interface, an interface compatible with a mobile communication standard such as LTE, 4G standard, or 5G standard, or an interface compatible with a short-range wireless communication standard such as Bluetooth (registered trademark). The term "LTE" is an abbreviation for long term evolution. The term "4G" is an abbreviation for 4th generation. The term "5G" is an abbreviation for 5th generation. The communication unit 23 receives the data used for the operation of the arterial-pressure estimation apparatus 20 and transmits the data obtained by the operation of the arterial-pressure estimation apparatus 20.

The input unit 24 includes at least one input interface. The input interface is, for example, a physical key, a capacitive key, a pointing device, a touch screen provided integrally with a display, an image-capturing device such as a camera, or a microphone. The input unit 24 receives an operation of inputting the data used for the operation of the arterial-pressure estimation apparatus 20. The input unit 24 may be connected to the arterial-pressure estimation apparatus 20 as an external input device instead of being provided in the arterial-pressure estimation apparatus 20. As a connection interface, for example, an interface compatible with a standard such as USB, HDMI (registered trademark), or Bluetooth (registered trademark) can be used. The term "USB" is an abbreviation for universal serial bus. The term "HDMI (registered trademark)" is an abbreviation for High-Definition Multimedia Interface.

The output unit 25 includes at least one output interface. The output interface is, for example, a display or a speaker. The display is, for example, an LCD or an organic EL display. The term "LCD" is an abbreviation for liquid crystal display. The term "EL" is an abbreviation for electro luminescence. The output unit 25 outputs the data obtained by the operation of the arterial-pressure estimation apparatus 20. The output unit 25 may be connected to the arterial-pressure estimation apparatus 20 as an external output device instead of being provided in the arterial-pressure estimation apparatus 20. As a connection interface, for example, an interface compatible with a standard such as USB, HDMI (registered trademark), or Bluetooth (registered trademark) can be used.

A function of the arterial-pressure estimation apparatus 20 is implemented by executing a program according to the present embodiment by a processor as the control unit 21. That is, the function of the arterial-pressure estimation apparatus 20 is implemented by software. The program causes a computer to function as the arterial-pressure estimation apparatus 20 by causing the computer to execute the operation of the arterial-pressure estimation apparatus 20. That is, the computer functions as the arterial-pressure estimation apparatus 20 by executing the operation of the arterial-pressure estimation apparatus 20 according to the program.

The program may be stored in a non-transitory computer-readable medium in advance. The non-transitory computer-readable medium is, for example, a flash memory, a magnetic recording device, an optical disc, a magnetooptical recording medium, or a ROM. Distribution of the program is executed by, for example, selling, transferring, or lending a portable medium such as an SD card, a DVD, or a CD-ROM storing the program. The term "SD" is an abbreviation for secure digital. The term "DVD" is an abbreviation for digital versatile disc. The term "CD-ROM" is an abbreviation for compact disc read only memory. The program may be distributed by storing the program in a storage of a server in advance and transferring the program from the server to another computer. The program may be provided as a program product.

For example, the computer temporarily stores, in the main storage device, the program stored in the portable medium or the program transferred from the server. The computer reads, by the processor, the program stored in the main storage device, and executes, by the processor, a process according to the read program. The computer may read the program directly from the portable medium and execute the process according to the program. Each time the program is transferred from the server to the computer, the computer may sequentially execute a process according to the received program. The process may be executed by a so-called ASP type service in which the function is implemented only by execution instruction and result acquisition without transferring the program from the server to the computer. The term "ASP" is an abbreviation for application service provider. The program includes information provided for a process by an electronic computer and conforming to the program. For example, data that is not a direct command to the computer but has a property of defining the process of the computer corresponds to the "information conforming to the program".

The functions of the arterial-pressure estimation apparatus 20 may be partially or entirely implemented by the programmable circuit or the dedicated circuit as the control unit 21. That is, the functions of the arterial-pressure estimation apparatus 20 may be partially or entirely implemented by hardware.

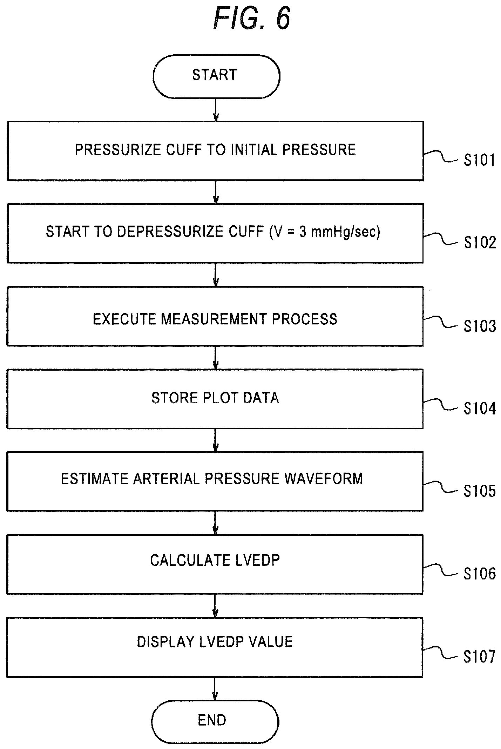

An operation of the arterial-pressure estimation system 10 according to the present embodiment will be described with reference to Fig. 6. The operation corresponds to an arterial-pressure estimation method according to the present embodiment.

In step S101, the cuff control apparatus 30 pressurizes the cuff 31 to an initial pressure. The initial pressure may be substantially the same as an initial pressure in a general non-invasive blood pressure measurement. The process of step S101 may be triggered by a command from the arterial-pressure estimation apparatus 20 to the cuff control apparatus 30.

In step S102, the cuff control apparatus 30 starts to depressurize the cuff 31. The cuff control apparatus 30 gradually reduces a cuff pressure at a constant depressurization speed V. The depressurization speed V may be any speed and is 3 mmHg/sec in the present embodiment. The process of step S102 may be triggered by a command from the arterial-pressure estimation apparatus 20 to the cuff control apparatus 30.

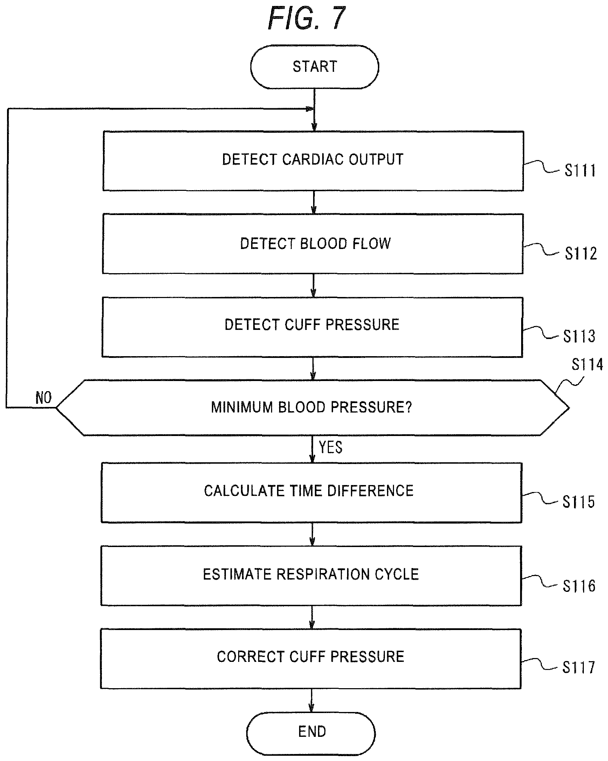

In step S103, a measurement process shown in Fig. 7 is executed.

In step S111, the cardiac output sensor 40 detects a cardiac output of the heart 11. Specifically, the cardiac output sensor 40 measures an electrocardiographic waveform. The cardiac output sensor 40 outputs a signal indicating the measured electrocardiographic waveform.

In step S112, the blood flow sensor 42 detects a blood flow generated at a location of the blood vessel 12 downstream of the aorta 18 compressed by the cuff 31. Specifically, the blood flow sensor 42 detects a K sound generated by the blood flow breaking through the cuff 31. The blood flow sensor 42 outputs a signal indicating a waveform of the K sound.

In step S113, the cuff pressure sensor 41 detects the cuff pressure as the pressure Pi. The cuff pressure sensor 41 outputs a signal indicating the pressure Pi.

In step S114, the control unit 21 of the arterial-pressure estimation apparatus 20 acquires the sensor data 44. The sensor data 44 includes data indicating, as the cardiac output timing Ti, a timing at which the cardiac output is detected in step S111 for the heartbeat i. Specifically, the sensor data 44 includes, as such data, data indicating the electrocardiographic waveform measured by the cardiac output sensor 40. The sensor data 44 further includes data indicating, as the blood flow timing Fi, a timing at which the blood flow is detected in step S112 for the heartbeat i. Specifically, the sensor data 44 includes, as such data, data indicating the waveform of the K sound detected by the blood flow sensor 42. The sensor data 44 further includes a value of the pressure Pi detected in step S113 for the heartbeat i. The control unit 21 stores the sensor data 44 in the storage unit 22, thereby recording the value of the pressure Pi in association with the blood flow timing Fi. The control unit 21 determines whether the pressure Pi is equal to or less than the minimum blood pressure. The minimum blood pressure may be specified by using any method, and in the present embodiment, the minimum blood pressure is specified by a blood pressure monitor using a general method, such as an oscillometric method or a Riva-Rocci and Korotkoff method. For example, the minimum blood pressure can be specified by detecting a timing at which a K sound generated during depressurization disappears. If the pressure Pi is equal to or less than the minimum blood pressure, processes of steps S115 to S117 are executed. If the pressure Pi is not equal to or less than the minimum blood pressure, the processes of steps S111 to S114 are executed again.

In step S115, the control unit 21 of the arterial-pressure estimation apparatus 20 calculates the time difference Di between the cardiac output timing Ti and the blood flow timing Fi indicated by the sensor data 44. Specifically, the control unit 21 specifies the cardiac output timing Ti based on the electrocardiographic waveform measured by the cardiac output sensor 40. The control unit 21 specifies, as the blood flow timing Fi, a timing at which the blood breaks through the cuff 31 based on the waveform of the K sound detected by the blood flow sensor 42. The control unit 21 calculates a difference between the specified timings as the time difference Di.

The cardiac output timing Ti may be specified based on a heart sound instead of the electrocardiographic waveform. In this case, in step S111, the cardiac output sensor 40 detects the heart sound including the choking sound of the mitral valve. The cardiac output sensor 40 outputs a signal indicating the detected heart sound. In step S114, the sensor data 44 includes data indicating the heart sound detected by the cardiac output sensor 40. The control unit 21 of the arterial-pressure estimation apparatus 20 specifies the cardiac output timing Ti based on the heart sound detected by the cardiac output sensor 40.

In step S116, the control unit 21 of the arterial-pressure estimation apparatus 20 estimates a respiration cycle. Specifically, the control unit 21 estimates the respiration cycle based on the electrocardiographic waveform measured by the cardiac output sensor 40 in step S111 by using a method disclosed in JP2018-011753A or any other method. Alternatively, the control unit 21 may estimate the respiration cycle based on the heart sound by using a method disclosed in JP2012-249730A or any other method. Alternatively, the control unit 21 may estimate the respiration cycle based on a pulse pressure or using a PPG or a millimeter wave radar.

In step S117, the control unit 21 of the arterial-pressure estimation apparatus 20 corrects, among the values of the pressures P1, P2, ..., and Pn recorded in step S114, a value of a pressure corresponding to a blood flow timing included in an inspiration period of the respiration cycle estimated in step S116. Specifically, the control unit 21 corrects the value of the pressure corresponding to the blood flow timing included in the inspiration period by performing weighting according to an elapsed time in the inspiration period. That is, the control unit 21 corrects a blood pressure value measured at the time of inspiration with a value calculated by a weighting coefficient that matches a respiration waveform. In the present embodiment, the control unit 21 corrects a value of the cuff pressure by adding a correction value y obtained according to the following equation to a value of a cuff pressure at the time of inspiration.

$$y = A \cdot \sin(2\pi f t)$$

A weighting coefficient A corresponds to a correction value width. The weighting coefficient A is set to, for example, 10 mmHg. The control unit 21 may set a weighting coefficient based on waveform data indicating a blood pressure waveform obtained by performing an invasive examination. That is, the correction value width may be determined based on the blood pressure waveform obtained by performing the invasive examination. In this case, the input unit 24 of the arterial-pressure estimation apparatus 20 may be used as an interface for inputting a blood pressure waveform. The weighting may be optimized by machine learning. That is, a trained model that receives inputs of the blood pressure waveform and the respiration cycle and outputs the weighting coefficient A may be constructed, and the weighting coefficient A may be determined using the trained model. A frequency f corresponds to a reciprocal of the respiration cycle. A time t is an elapsed time from the start of the inspiration to the blood flow timing. For example, when A = 10 mmHg, f = 0.2 Hz, and t = 0.5 seconds, y = 10 sin(2$\pi$ × 0.2 × 0.5) ≈ 5.9 mmHg.

In step S104, the control unit 21 of the arterial-pressure estimation apparatus 20 generates plot data by referring to the sensor data 44 acquired in step S103 and the value of the pressure recorded and corrected in step S103. Specifically, for each heartbeat i, the control unit 21 plots the time difference Di calculated in step S115, a value of the pressure Pi after correction if the value of the pressure Pi is corrected in step S117, and a value of the pressure Pi before correction if the value of the pressure Pi is not corrected, that is, the value of the pressure Pi recorded in step S114. The control unit 21 stores the obtained plot data in the storage unit 22.

In step S105, the control unit 21 of the arterial-pressure estimation apparatus 20 estimates a change in an arterial pressure over time based on the plot data obtained in step S104. That is, the control unit 21 estimates the change in the arterial pressure over time according to the time difference Di between the cardiac output timing Ti and the blood flow timing Fi indicated by the sensor data 44 and the value of the pressure Pi indicated by the sensor data 44. Specifically, the control unit 21 performs spline interpolation on plotted points to estimate a curve of the arterial pressure waveform 15.

In step S106, the control unit 21 of the arterial-pressure estimation apparatus 20 estimates an LVEDP based on an estimation result of the change in the arterial pressure over time obtained in step S105. Specifically, the control unit 21 estimates a left ventricular pressure waveform according to the arterial pressure waveform 15 estimated in step S105. As a method of estimating a left ventricular pressure waveform, for example, a method similar to that in the comparative example can be used. The control unit 21 acquires an estimated value of the LVEDP based on the estimated left ventricular pressure waveform.

In step S107, the control unit 21 of the arterial-pressure estimation apparatus 20 presents the estimated value of the LVEDP acquired in step S106 to the user. Specifically, the control unit 21 displays the estimated value of the LVEDP on the display as the output unit 25. Alternatively, the control unit 21 may output the estimated value of the LVEDP by voice from the speaker as the output unit 25. Alternatively, the control unit 21 may cause the communication unit 23 to transmit the estimated value of the LVEDP. The communication unit 23 may transmit the estimated value of the LVEDP to a mobile device such as a mobile phone, a smartphone, or a tablet, or a terminal device of the user such as a PC, directly or via a network such as a LAN or the Internet, and cause the terminal device to present the estimated value of the LVEDP.

As described above, in the present embodiment, the control unit 21 of the arterial-pressure estimation apparatus 20 acquires the sensor data 44 indicating, as the blood flow timing, the timing at which the blood flow generated at the one or more locations of the blood vessel 12 downstream of the aorta 18 is detected for each heartbeat when the one or more locations is compressed while the pressure is gradually reduced, and records the value of the pressure corresponding to each blood flow timing. The control unit 21 corrects the value of the pressure corresponding to the blood flow timing included in the inspiration period of the respiration cycle. The control unit 21 estimates the change in the arterial pressure over time by referring to the acquired sensor data 44 and the recorded and corrected value of the pressure.

According to the present embodiment, the influence of the respiratory variation can be reduced. Therefore, the change in the arterial pressure over time can be noninvasively and highly accurately estimated.

As shown in Fig. 1, the arterial-pressure estimation system 10 includes at least one cuff 31, the cuff control apparatus 30, the blood flow sensor 42 such as a K-sound sensor, a pulse pressure sensor, a PPG sensor, or an ultrasonic Doppler sensor, the cuff pressure sensor 41, the cardiac output sensor 40 such as the ECG sensor or a heart sound sensor, and the arterial-pressure estimation apparatus 20.

The cuff 31 compresses the blood vessel 12 to occlude the blood vessel 12. The cuff control apparatus 30 pressurizes the cuff 31 to the initial pressure and depressurizes the cuff 31. The blood flow sensor 42 detects a timing at which a blood flow is generated during the depressurization of the cuff 31. The cuff pressure sensor 41 detects a cuff pressure at the timing at which the blood flow is generated. The cardiac output sensor 40 detects a cardiac output timing of the heart 11.

The arterial-pressure estimation apparatus 20 estimates a respiration cycle based on an ECG or a heart sound. The arterial-pressure estimation apparatus 20 corrects a cuff pressure value obtained for each beat according to the respiration cycle. The arterial-pressure estimation apparatus 20 calculates a time difference between the cardiac output timing of the heart 11 and the timing at which the blood flow is generated. The arterial-pressure estimation apparatus 20 estimates at least a part of the arterial pressure waveform 15 based on the time difference and the cuff pressure value after correction.

According to the present embodiment, the reproducibility of the estimated arterial pressure waveform 15 can be improved.

As a modification of the present embodiment, the control unit 21 of the arterial-pressure estimation apparatus 20 may detect a respiration cycle by measuring a respiration flow or using a belt-type respiration sensor wound around a chest or an abdomen instead of estimating the respiration cycle. Alternatively, the control unit 21 may instruct a timing of respiration when a measurement process is executed or may forcibly control respiration in conjunction with a CPAP device or an artificial respirator. The term "CPAP" is an abbreviation for continuous positive airway pressure. If there is no need to estimate or detect the respiration cycle, the measurement process can be speeded up.

As a modification of the present embodiment, the control unit 21 of the arterial-pressure estimation apparatus 20 may input the plot data obtained in step S104 to the trained model and estimate an LVEDP using the trained model instead of estimating the curve of the arterial pressure waveform 15. That is, the control unit 21 may input an estimation result of a change in an arterial pressure over time to the trained model and acquire an estimated value of the LVEDP from the trained model.

As a modification of the present embodiment, the control unit 21 of the arterial-pressure estimation apparatus 20 may construct a trained model for estimating an LVEDP by using at least a part of the arterial pressure waveform 15. For example, the control unit 21 may construct a trained model in which the maximum slope of rise of the arterial pressure waveform 15 is a part of an explanatory variable. When estimating an LVEDP in the next and subsequent times, the control unit 21 may estimate the LVEDP using the trained model. That is, the control unit 21 may generate a trained model for acquiring an estimated value of the LVEDP by performing machine learning using at least a part of an estimation result of a change in an arterial pressure over time. The control unit 21 may store the generated trained model in the storage unit 22 such that the trained model is usable in the future.

As a modification of the present embodiment, the measurement process may be executed multiple times to efficiently collect the plot data. Such a modification will be described.

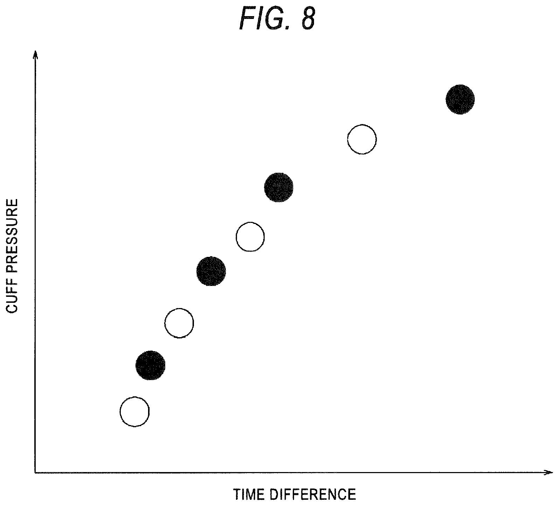

In the modification, the number of plots forming a waveform can be increased as shown in Fig. 8 by matching a depressurization start timing with a respiration cycle, repeating remeasurement continuously, and changing a depressurization speed each time.

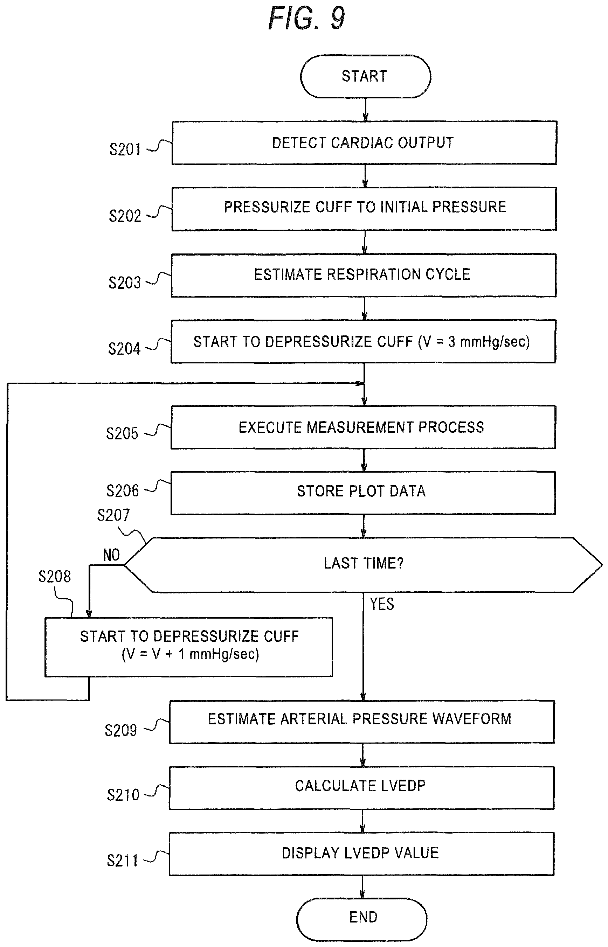

An operation of the arterial-pressure estimation system 10 according to the modification will be described with reference to Fig. 9. The operation corresponds to an arterial-pressure estimation method according to the modification.

In step S201, the cardiac output sensor 40 starts to detect a cardiac output of the heart 11, and then repeatedly detects the cardiac output of the heart 11. Specifically, the cardiac output sensor 40 repeatedly measures an electrocardiographic waveform. The cardiac output sensor 40 outputs a signal indicating the electrocardiographic waveform each time the electrocardiographic waveform is measured.

Since a process of step S202 is the same as the process of step S101, the description of the process of S202 is omitted.

In step S203, the control unit 21 of the arterial-pressure estimation apparatus 20 estimates a respiration cycle. Specifically, the control unit 21 estimates the respiration cycle based on the electrocardiographic waveform measured by the cardiac output sensor 40 in step S201 by using the same method as in step S116. Alternatively, the control unit 21 may estimate the respiration cycle based on a heart sound or a pulse pressure, or using a PPG or a millimeter wave radar.

In step S204, the cuff control apparatus 30 starts to depressurize the cuff 31. The cuff control apparatus 30 gradually reduces a cuff pressure at a constant depressurization speed V. The depressurization speed V may be any speed and is 3 mmHg/sec in the modification as well.

In the modification, the process of step S204 is triggered by a command from the arterial-pressure estimation apparatus 20 to the cuff control apparatus 30. Specifically, the control unit 21 of the arterial-pressure estimation apparatus 20 causes the cuff control apparatus 30 to start the depressurization in accordance with an expiration timing in the respiration cycle estimated in step S203.

Since processes of steps S205 and S206 are the same as the processes of steps S103 and S104, the description of S205 and S206 is omitted.

In step S207, the control unit 21 of the arterial-pressure estimation apparatus 20 determines whether the number of times the measurement process is executed reaches a specified number. The specified number may be any number of times of 2 or more, and is 3 times in the modification. If the number of times the measurement process is executed reaches the specified number, processes of steps S209 to S211 are executed. If the number of times the measurement process is executed does not reach the specified number, a process of step S208 is executed.

In step S208, the cuff control apparatus 30 pressurizes the cuff 31 to the initial pressure again and then starts to depressurize the cuff 31. Similar to step S202, the cuff control apparatus 30 gradually reduces the cuff pressure at a constant depressurization speed V. The depressurization speed V is a speed faster than that when the measurement process is executed last time, and in the modification, the depressurization speed V is a speed 1 mmHg/sec faster than the last speed. That is, the depressurization speed V is 4 mmHg/sec when the measurement process is executed for a second time, and is 5 mmHg/sec when the measurement process is executed for a third time. The process of step S208 is triggered by a command from the arterial-pressure estimation apparatus 20 to the cuff control apparatus 30, similar to the process of step S202. Specifically, the control unit 21 of the arterial-pressure estimation apparatus 20 causes the cuff control apparatus 30 to start the depressurization in accordance with an expiration timing in a recently estimated respiration cycle. After step S208, the processes of steps S205 to S207 are executed again.

Since the processes of steps S209 to S211 are the same as the processes of steps S105 to S107, the description of S209 to S211 is omitted.

As described above, in the modification, the cuff 31 compresses one location of the blood vessel 12 multiple times while the cuff pressure is reduced at different speeds each time under the control of the cuff control apparatus 30. The sensor data 44 includes data indicating, as a blood flow timing, a timing at which a blood flow is detected for each heartbeat in each of the multiple times. The control unit 21 of the arterial-pressure estimation apparatus 20 performs control to match a timing at which a pressure starts to be reduced with the respiration cycle.

According to the modification, by increasing the number of samples, the change in the arterial pressure over time can be estimated with higher accuracy.

As a further modification of the modification, for each remeasurement, a different depressurization speed may be applied each time until a blood flow is detected for a first time, and the same depressurization speed may be applied each time after a blood flow is detected. That is, in the process of step S205 executed after the process of step S208, a depressurization speed set in the process of step S208 immediately before a blood flow timing F1 may be applied, and a depressurization speed set in the process of step S204 after the blood flow timing F1 may be applied. For example, in step S208, the cuff control apparatus 30 pressurizes the cuff 31 to the initial pressure again, and then starts to depressurize the cuff 31 at a speed 1 mmHg/sec faster than the last speed. Then, in step S205, after the first step S112, that is, once the blood flow is detected, the cuff control apparatus 30 gradually reduces the cuff pressure at the same speed as the first time.

As described above, the cuff 31 may compress one location of the blood vessel 12 multiple times while the cuff pressure starts to be reduced at different speeds each time and is reduced at a common speed after a heartbeat at which a blood flow is detected under the control of the cuff control apparatus 30.

The present disclosure is not limited to the above-described embodiments. For example, two or more blocks described in a block diagram may be integrated, or one block may be divided. Instead of executing two or more steps described in the flowchart in time series according to the description, the steps may be executed in parallel or in a different order according to a process capability of the apparatus that executes each step or as necessary. In addition, modifications can be made without departing from a gist of the present disclosure.

For example, the control unit 21 of the arterial-pressure estimation apparatus 20 may estimate a hemodynamic parameter other than the LVEDP based on the estimation result of the change in the arterial pressure over time. The hemodynamic parameter is a parameter related to intracardiac hemodynamics, such as an LVEDP, a pulmonary artery pressure, or a pulmonary wedge pressure. The pulmonary artery pressure is also abbreviated as "PAP". The pulmonary wedge pressure is also abbreviated as "PWP", is also referred to as pulmonary arterial wedge pressure or abbreviated as "PAWP", is also referred to as pulmonary capillary wedge pressure or abbreviated as "PCWP", or is also referred to as pulmonary artery occlusion pressure or abbreviated as "PAOP". The control unit 21 may generate a trained model for acquiring an estimated value of the hemodynamic parameter other than the LVEDP by performing machine learning using at least a part of the estimation result of the change in the arterial pressure over time. The control unit 21 may present the estimated value of the hemodynamic parameter other than the LVEDP to the user.

In a specific example in which a pulmonary artery pressure is estimated as a hemodynamic parameter, the control unit 21 of the arterial-pressure estimation apparatus 20 estimates an arterial pressure waveform as a change in an arterial pressure over time. The control unit 21 estimates a pulmonary artery pressure waveform according to the estimated arterial pressure waveform. The control unit 21 acquires an estimated value of the pulmonary artery pressure based on the estimated pulmonary artery pressure waveform. Alternatively, the control unit 21 may input an estimation result of the change in the arterial pressure over time to the trained model and acquire an estimated value of the pulmonary artery pressure from the trained model instead of estimating the pulmonary artery pressure waveform.

In a specific example in which a pulmonary wedge pressure is estimated as a hemodynamic parameter, the control unit 21 of the arterial-pressure estimation apparatus 20 estimates an arterial pressure waveform as a change in an arterial pressure over time. The control unit 21 estimates a pulmonary wedge pressure waveform, a right atrial pressure waveform, or a right ventricular pressure waveform according to the estimated arterial pressure waveform. The control unit 21 acquires an estimated value of the pulmonary wedge pressure based on the estimated pulmonary wedge pressure waveform, right atrial pressure waveform, or right ventricular pressure waveform. Alternatively, the control unit 21 may input an estimation result of the change in the arterial pressure over time to the trained model and acquire an estimated value of the pulmonary wedge pressure from the trained model, instead of estimating the pulmonary wedge pressure waveform, the right atrial pressure waveform, or the right ventricular pressure waveform.

Reference Signs List

10: arterial-pressure estimation system
11: heart

12: blood vessel
13: arm
15: arterial pressure waveform
16: left ventricle
17: aortic valve
18: aorta
20: arterial-pressure estimation apparatus
21: control unit
22: storage unit
23: communication unit
24: input unit
25: output unit
30: cuff control apparatus
31: cuff
40: cardiac output sensor
41: cuff pressure sensor
42: blood flow sensor
44: sensor data
90: cuff
91: first K-sound microphone
92: second K-sound microphone
93: pressure sensor

Claims

1. An arterial-pressure estimation apparatus comprising:
   a control unit configured to acquire sensor data indicating, as a blood flow timing, a timing at which a blood flow generated at one or more locations of a blood vessel downstream of an aorta is detected for each heartbeat when the one or more locations is compressed while a pressure is gradually reduced, record a value of the pressure corresponding to each blood flow timing, correct the value of the pressure corresponding to the blood flow timing included in an inspiration period of a respiration cycle, and estimate a change in an arterial pressure over time by referring to the acquired sensor data and the recorded and corrected value of the pressure.

2. The arterial-pressure estimation apparatus according to claim 1, wherein
   the control unit corrects the value of the pressure corresponding to the blood flow timing included in the inspiration period by performing weighting according to an elapsed time in the inspiration period.

3. The arterial-pressure estimation apparatus according to claim 2, wherein
   the control unit sets a weighting coefficient based on waveform data indicating a blood pressure waveform obtained by performing an invasive examination.

4. The arterial-pressure estimation apparatus according to any one of claims 1 to 3, wherein

the sensor data includes data indicating, as the blood flow timing, a timing at which the one or more locations is compressed multiple times while the pressure is reduced at different speeds each time, and the blood flow is detected for each heartbeat in each of the multiple times, and the control unit performs control to match a timing at which the pressure starts to be reduced with the respiration cycle.

5. The arterial-pressure estimation apparatus according to any one of claims 1 to 3, wherein

the sensor data includes data indicating, as the blood flow timing, a timing at which the one or more locations is compressed multiple times while the pressure starts to be reduced at different speeds each time and is reduced at a common speed after a heartbeat at which the blood flow is detected, and the blood flow is detected for each heartbeat in each of the multiple times, and
the control unit performs control to match a timing at which the pressure starts to be reduced with the respiration cycle.

6. The arterial-pressure estimation apparatus according to claim 1, wherein

the sensor data includes data indicating, as a cardiac output timing, a timing at which a cardiac output is detected for each heartbeat, and
the control unit estimates a change in the arterial pressure over time according to a time difference between the cardiac output timing and the blood flow timing indicated by the sensor data and the value of the pressure.

7. The arterial-pressure estimation apparatus according to any one of claims 1 to 6, wherein
the control unit estimates an LVEDP based on an estimation result of the change in the arterial pressure over time.

8. The arterial-pressure estimation apparatus according to claim 7, wherein
the control unit estimates an arterial pressure waveform as the change in the arterial pressure over time, estimates a left ventricular pressure waveform according to the estimated arterial pressure waveform, and acquires an estimated value of the LVEDP based on the estimated left ventricular pressure waveform.

9. The arterial-pressure estimation apparatus according to claim 7, wherein
the control unit inputs the estimation result of the change in the arterial pressure over time to a trained model and acquires an estimated value of the LVEDP from the trained model.

10. The arterial-pressure estimation apparatus according to any one of claims 7 to 9, wherein
the control unit generates a trained model for acquiring an estimated value of the LVEDP by performing machine learning using at least a part of the estimation result of the change in the arterial pressure over time.

11. The arterial-pressure estimation apparatus according to any one of claims 7 to 10, wherein
the control unit presents an estimated value of the LVEDP to a user.

12. The arterial-pressure estimation apparatus according to any one of claims 1 to 6, wherein
the control unit estimates a parameter related to intracardiac hemodynamics based on an estimation result of the change in the arterial pressure over time.

13. The arterial-pressure estimation apparatus according to claim 12, wherein
the parameter includes a pulmonary artery pressure or a pulmonary wedge pressure.

14. An arterial-pressure estimation system comprising:

the arterial-pressure estimation apparatus according to any one of claims 1 to 13; and
a sensor configured to detect the blood flow.

15. The arterial-pressure estimation system according to claim 14 further comprising:
an inflation portion configured to compress the at least one location.

16. An arterial-pressure estimation method comprising:

an inflation portion compressing one or more locations of a blood vessel downstream of an aorta while a pressure is gradually reduced;
a sensor detecting, for each heartbeat, a blood flow generated at the one or more locations;
a control unit acquiring sensor data indicating, as a blood flow timing, a timing at which the blood flow is detected for each heartbeat when the one or more locations is compressed;
the control unit recording a value of the pressure corresponding to each blood flow timing;
the control unit correcting the value of the pressure corresponding to the blood flow timing included in an inspiration period of a respiration cycle; and
the control unit estimating a change in an arterial pressure over time by referring to the acquired sensor data and the recorded and corrected val-

**EP 4 285 815 A1**

ue of the pressure.

FIG. 1

*FIG. 2*

20

ARTERIAL-PRESSURE ESTIMATION APPARATUS

21

CONTROL UNIT

22

STORAGE UNIT

COMMUNICATION UNIT

23

INPUT UNIT

24

OUTPUT UNIT

25

EP 4 285 815 A1

FIG. 3

## FIG. 4

RESPIRATION INSPIRATION EXPIRATION

CUFF PRESSURE
BLOOD PRESSURE
WAVEFORM

K SOUND

TIME
DIFFERENCE

ECG

EP 4 285 815 A1

## FIG. 5

## FIG. 6

```
                    ┌──────────┐
                    │  START   │
                    └──────────┘
                         │
                         ▼
    ┌────────────────────────────────────────┐
    │   PRESSURIZE CUFF TO INITIAL PRESSURE   │────  S101
    └────────────────────────────────────────┘
                         │
                         ▼
    ┌────────────────────────────────────────┐
    │ START TO DEPRESSURIZE CUFF (V = 3 mmHg/sec) │──  S102
    └────────────────────────────────────────┘
                         │
                         ▼
    ┌────────────────────────────────────────┐
    │       EXECUTE MEASUREMENT PROCESS       │────  S103
    └────────────────────────────────────────┘
                         │
                         ▼
    ┌────────────────────────────────────────┐
    │            STORE PLOT DATA              │────  S104
    └────────────────────────────────────────┘
                         │
                         ▼
    ┌────────────────────────────────────────┐
    │   ESTIMATE ARTERIAL PRESSURE WAVEFORM   │────  S105
    └────────────────────────────────────────┘
                         │
                         ▼
    ┌────────────────────────────────────────┐
    │            CALCULATE LVEDP              │────  S106
    └────────────────────────────────────────┘
                         │
                         ▼
    ┌────────────────────────────────────────┐
    │          DISPLAY LVEDP VALUE            │────  S107
    └────────────────────────────────────────┘
                         │
                         ▼
                    ┌──────────┐
                    │   END    │
                    └──────────┘
```

## FIG. 7

START

DETECT CARDIAC OUTPUT ⸺ S111

DETECT BLOOD FLOW ⸺ S112

DETECT CUFF PRESSURE ⸺ S113

MINIMUM BLOOD PRESSURE? S114

NO

YES

CALCULATE TIME DIFFERENCE ⸺ S115

ESTIMATE RESPIRATION CYCLE ⸺ S116

CORRECT CUFF PRESSURE ⸺ S117

END

FIG. 8

# FIG. 9

```
                    ┌─────────────┐
                    │    START    │
                    └─────────────┘
                           │
                           ▼
S201   ┌──────────────────────────────────────────────┐
       │            DETECT CARDIAC OUTPUT              │
       └──────────────────────────────────────────────┘
                           │
                           ▼
S202   ┌──────────────────────────────────────────────┐
       │      PRESSURIZE CUFF TO INITIAL PRESSURE      │
       └──────────────────────────────────────────────┘
                           │
                           ▼
S203   ┌──────────────────────────────────────────────┐
       │           ESTIMATE RESPIRATION CYCLE         │
       └──────────────────────────────────────────────┘
                           │
                           ▼
S204   ┌──────────────────────────────────────────────┐
       │    START TO DEPRESSURIZE CUFF (V = 3 mmHg/sec)│
       └──────────────────────────────────────────────┘
                           │
                           ▼
S205   ┌──────────────────────────────────────────────┐
       │           EXECUTE MEASUREMENT PROCESS        │
       └──────────────────────────────────────────────┘
                           │
                           ▼
S206   ┌──────────────────────────────────────────────┐
       │               STORE PLOT DATA                │
       └──────────────────────────────────────────────┘
                           │
                           ▼
S207             ◁────────────────────────────────────▷
         NO      │            LAST TIME?              │
                 ◁────────────────────────────────────▷
                           │ YES
S208   ┌────────────────────────────┐
       │  START TO DEPRESSURIZE CUFF │
       │     (V = V + 1 mmHg/sec)    │
       └────────────────────────────┘
                           │
                           ▼
S209   ┌──────────────────────────────────────────────┐
       │      ESTIMATE ARTERIAL PRESSURE WAVEFORM      │
       └──────────────────────────────────────────────┘
                           │
                           ▼
S210   ┌──────────────────────────────────────────────┐
       │               CALCULATE LVEDP                │
       └──────────────────────────────────────────────┘
                           │
                           ▼
S211   ┌──────────────────────────────────────────────┐
       │             DISPLAY LVEDP VALUE              │
       └──────────────────────────────────────────────┘
                           │
                           ▼
                    ┌─────────────┐
                    │     END     │
                    └─────────────┘
```

## FIG. 10A

## FIG. 10B

## FIG. 11A

## FIG. 11B

## FIG. 12A

ARTERIAL PRESSURE (VICINITY OF AORTIC VALVE)

OPEN AORTIC VALVE

OBJECTIVE FUNCTION: LVEDP

LEFT VENTRICULAR PRESSURE

DELAY

HEART SOUND

I SOUND (CLOSE MITRAL VALVE)

OUTFLOW SOUND

II SOUND (CLOSE AORTIC VALVE)

INFLOW SOUND

CUFF PRESSURE

ARTERIAL PRESSURE (UPPER ARM)

DELAY

## FIG. 12B

18 — AORTA

93

UPPER ARM — 13

91

92

90

17 — VALVE

16 — LEFT VENTRICLE

## FIG. 13A

ARTERIAL
PRESSURE
(VICINITY OF
AORTIC VALVE)

OPEN AORTIC
VALVE

OBJECTIVE
FUNCTION: LVEDP

DELAY
LEFT
VENTRICULAR
PRESSURE

OUTFLOW
SOUND

INFLOW
SOUND

HEART
SOUND
I SOUND
(CLOSE MITRAL
VALVE)

II SOUND
(CLOSE AORTIC
VALVE)

ARTERIAL
PRESSURE
(UPPER ARM)

CUFF
PRESSURE

DELAY

ELECTROCAR
DIOGRAPHIC
WAVEFORM

FIRST K-
SOUND
(UPPER ARM)

CUFF
PRESSURE

TIME INTERVAL FROM Q WAVE TO K SOUND

## FIG. 13B

18 — AORTA

93

UPPER ARM — 13

91

17 — VALVE

92

90

16 — LEFT
VENTRICLE

26

## FIG. 14

# FIG. 15

ARTERIAL PRESSURE (VICINITY OF AORTIC VALVE)

OPEN AORTIC VALVE

OBJECTIVE FUNCTION: LVEDP

DELAY

LEFT VENTRICULAR PRESSURE

OUTFLOW SOUND

INFLOW SOUND

HEART SOUND

I SOUND (CLOSE MITRAL VALVE)

II SOUND (CLOSE AORTIC VALVE)

ARTERIAL PRESSURE (UPPER ARM)

CUFF PRESSURE

DELAY

ELECTROCAR DIOGRAPHIC WAVEFORM

FIRST K-SOUND (UPPER ARM)

CUFF PRESSURE

FITTED CURVE

TIME INTERVAL FROM Q WAVE TO K SOUND

# FIG. 16

TIME INTERVAL FROM Q WAVE TO K SOUND

## INTERNATIONAL SEARCH REPORT

| International application No. |
| --- |
| **PCT/JP2022/003892** |

**A.  CLASSIFICATION OF SUBJECT MATTER**

*A61B 5/022*(2006.01)i; *A61B 5/0205*(2006.01)i; *A61B 5/0215*(2006.01)i
FI:   A61B5/022 400M; A61B5/0205; A61B5/0215 A

According to International Patent Classification (IPC) or to both national classification and IPC

**B.  FIELDS SEARCHED**

Minimum documentation searched (classification system followed by classification symbols)

A61B5/022; A61B5/0205; A61B5/0215

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
| --- | --- | --- |
| A | JP 2016-137087 A (NIPPON KODEN KOGYO KK) 04 August 2016 (2016-08-04)<br>entire text, all drawings | 1-16 |
| A | JP 2010-522621 A (CARDIAC PACEMAKERS, INC) 08 July 2010 (2010-07-08)<br>entire text, all drawings | 1-16 |
| A | US 2020/0359912 A1 (CALIFORNIA INSTITUTE OF TECHNOLOGY) 19 November 2020 (2020-11-19)<br>entire text, all drawings | 1-16 |
| A | JP 2015-160082 A (UNIV HIROSHIMA, NIPPON KODEN KOGYO KK) 07 September 2015 (2015-09-07)<br>entire text, all drawings | 1-16 |
| A | JP 2008-536570 A (MCINTYRE, Kevin M.) 11 September 2008 (2008-09-11)<br>entire text, all drawings | 1-16 |
| A | JP 2000-333911 A (NIPPON COLIN CO LTD) 05 December 2000 (2000-12-05)<br>entire text, all drawings | 1-16 |

☐ Further documents are listed in the continuation of Box C.          ☑ See patent family annex.

| | |
| --- | --- |
| *    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
| --- | --- |
| **15 April 2022** | **26 April 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
**Information on patent family members**

International application No.

**PCT/JP2022/003892**

| Patent document cited in search report | | | Publication date (day/month/year) | Patent family member(s) | | | Publication date (day/month/year) |
|---|---|---|---|---|---|---|---|
| JP | 2016-137087 | A | 04 August 2016 | US | 2016/0213332 | A1 | |
| | | | | entire text, all drawings | | | |
| | | | | EP | 3050500 | A1 | |
| JP | 2010-522621 | A | 08 July 2010 | US | 2008/0243016 | A1 | |
| | | | | entire text, all drawings | | | |
| | | | | WO | 2008/118951 | A1 | |
| US | 2020/0359912 | A1 | 19 November 2020 | WO | 2019/144058 | A1 | |
| | | | | entire text, all drawings | | | |
| JP | 2015-160082 | A | 07 September 2015 | US | 2015/0245772 | A1 | |
| | | | | entire text, all drawings | | | |
| | | | | EP | 2912996 | A1 | |
| | | | | CN | 104873182 | A | |
| JP | 2008-536570 | A | 11 September 2008 | US | 2006/0235309 | A1 | |
| | | | | entire text, all drawings | | | |
| | | | | WO | 2006/113366 | A2 | |
| | | | | CA | 2605146 | A1 | |
| JP | 2000-333911 | A | 05 December 2000 | US | 6334849 | B1 | |
| | | | | entire text, all drawings | | | |
| | | | | EP | 1055395 | A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150265163 A
- JP 2016137087 A
- JP H01214338 A
- JP 2018011753 A
- JP 2012249730 A